(19) Europäisches Patentamt
European Patent Office
Office européen des brevets

(11) Publication number: **0 078 434**
**B1**

(12) # EUROPEAN PATENT SPECIFICATION

(45) Date of publication of patent specification: **17.04.85**

(21) Application number: **82109581.7**

(22) Date of filing: **03.03.81**

(60) Publication number of the earlier application in accordance with Art. 76 EPC: **0 035 856**

(51) Int. Cl.[4]: **A 61 K 31/585,** A 61 K 33/30, A 61 K 45/02 // (A61K31/585, 31:20),(A61K31/585, 31:23)

(54) **Pharmaceutical and dietary compositions.**

(30) Priority: **07.03.80 GB 8007870**

(43) Date of publication of application:
**11.05.83 Bulletin 83/19**

(45) Publication of the grant of the patent:
**17.04.85 Bulletin 85/16**

(84) Designated Contracting States:
**AT BE CH DE FR GB IT LI LU NL SE**

(56) References cited:
**EP-A-0 003 407**
**EP-A-0 004 770**
**EP-A-0 019 423**
**EP-A-0 037 175**
**GB-A-1 082 624**

(73) Proprietor: **EFAMOL LIMITED**
**71/74 Mark Lane**
**London E.C.3 (GB)**

(72) Inventor: **Horrobin, David Frederick**
**P.O. Box 10**
**Nuns'Island Montreal H3E 1J8 (CA)**

(74) Representative: **Caro, William Egerton et al**
**J. MILLER & CO. Lincoln House 296-302 High Holborn**
**London WC1V 7JH (GB)**

**EP 0 078 434 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

FIELD OF THE INVENTION

This invention relates to the treatment of certain diseases and disorders primarily, but not exclusively, in the field of human medicine and to compositions for use therein.

GENERAL BACKGROUND

Considerable interest has been shown in recent years in the use of prostaglandin (PG) precursors in medicine.

For various reasons it is not practical to administer naturally-occurring prostaglandins such as PGE 1 and PGE 2 to patients. Consequently, considerable attention has focussed on the use of prostaglandin precursors including linoleic acid, γ-linolenic acid (GLA) and dihomo-γ-linolenic acid (DGLA).

Conversion of these materials in the body is believed to be as shown in the following diagram:

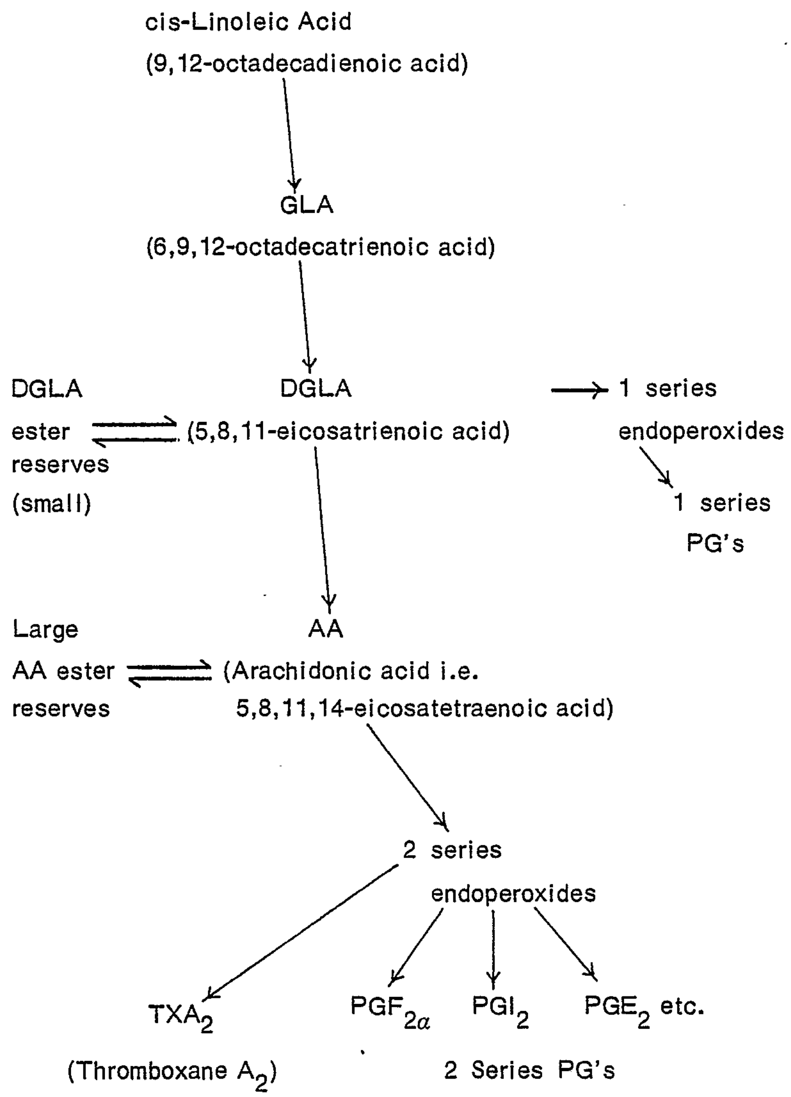


The broad outline of this pathway is well known, and it brings out clearly that a major function of essential fatty acids (EFAs) is to act as precursors for prostaglandins, 1-series PGs being formed from dihomo-γ-linolenic acid (DGLA) and 2-series PGs from arachidonic acid (AA). DGLA and AA are present in food in only small quantities, and the major EFA in food is linoleic acid which is first converted to γ-linolenic acid (GLA) and then to DGLA and AA. The conversion of linoleic acid to GLA is blocked by a high fat and high carbohydrate diet, by ageing and for example by diabetes. Stores of AA in the body in the form of lipid esters are very large indeed. In contrast only small amounts of DGLA esters are present.

INVENTION AND BACKGROUND

DGLA is the key substance. GLA is almost completely and very rapidly converted in the body to DGLA and so for practical purposes the oral administration of DGLA and GLA amounts to the same thing. DGLA

can be converted to a storage form, changed to arachidonic acid and thence to PGs of the 2-series, or converted to PGs of the 1-series.

There is increasing evidence that PGs of the 1-series play a vital role in a number of key areas. First, PGE 1 activates T lymphocytes. Defective T lymphocytes are believed to be involved in causing a wide range of allergic and inflammatory disorders and in making individuals susceptible to cancer and infections of all types. Second, PGE 1 is important in preventing over-production of collagen and fibrous tissue, a factor which plays a major role in arthritis and the so-called collagen diseases. Third, PGE 1 levels are extremely low in patients with schizophrenia and are moderately low in patients with depression. Fourth, PGE 1 appears to be important in controlling cholesterol levels and necessary for the normal actions of insulin. Fifth, PGE 1 dilates blood vessels and may be expected to be helpful in any situation in which vessel spasm occurs. Sixth, PGE 1 appears to inhibit the production of 2-series PGs, levels of which are raised in a wide variety of inflammatory disorders. Seventh, PGE 1 increases production of cyclic AMP which has antiinflammatory effects.

There are therefore very strong reasons, and this is the broad feature of the present invention, for influencing the 1-series/2-series PG balance in the body in favour of 1-series PGs and specifically for selectively enhancing formation of PGs of the 1-series and particularly PGE 1. The diseases and disorders below are among those in which such action is indicated:

1. Situations in which defective T lymphocyte function has been described such as allergic and inflammatory disorders, multiple sclerosis, schizophrenia and cancer.

2. Situations in which regulation of collagen formation and breakdown is defective including rheumatoid arthritis, systemic lupus erythematosus, Crohn's disease and the various "collagen" diseases.

3. Mental illnesses in which low PGE 1 levels have been reported, including depression and shcizophrenia. In depression, platelet PGE 1 production is moderately reduced whereas in schizophrenia it is severely reduced.

4. Disorders of lipid and carbohydrate metabolism in particular diabetes mellitus and situations in which blood cholesterol levels are elevated.

5. Disorders in which there is a tendency of blood vessels to go into spasm such as angina pectoris, myocardial infarction and Reynaud's syndrome.

Selective enhancement of 1-series PG production has been explored in human platelets. The method is given in detail later herein but briefly human platelets are incubated with radioactive DGLA or arachidonic acid. The PGs produced during incubation are extracted by conventional means and separated by thin layer chromatography, and the amount of radioactivity appearing in each PG or related substance is counted. PGE 1, PGF 1α and thromboxane B1 from DGLA, and PGE 2, PFG 2α and thromboxane B2 from AA are estimated. The results, as given herein, demonstrate the inventor's belief that the effects of various agents on AA and DGLA conversion can be quite different and that it is possible to selectively enhance formation of PGE 1 and other 1-series PG compounds. The effect is believed to be by influencing the conversion of DGLA to the 1-series PGs.

The balance between 1-series and 2-series PGs is, the inventor believes, significant in terms of overall control of the conversion pathways given earlier. Such control is not understood in detail but without restriction to the theory it appears first that PGE 2 is able to enhance the formation of 1-series PGs, and second that PGE 1 is able to block arachidonic acid mobilisation from tissue stores. Thus the conditions for a negative feedback control loop exist; overproduction of PGE 2 from AA will activate PGE 1 synthesis, the PGE 1 will inhibit AA mobilisation, and production of 2-series PGs will drop. Further, TXA 2, an unstable product of the 2-series endoperoxides arising in 2-series PG production, also appears to enhance 1-series PG and in particular PGE 1 production. Thus again the activity of the 2-series PG synthesis pathway gives rise indirectly to a material that controls that pathway.

EFFECTIVE AGENTS

Thus the combination of DGLA or GLA or their esters from any natural or synthetic source with spironolactone and compounds which have a steroid ring structure and which when tested on human platelets enhanced the conversion of DGLA (5,8,11-eicosatrienoic acid) to 1-series prostagalandins is proposed for use in any condition calling for selective enhancement of 1-series PG production or more broadly for influence of the 1-series/2-series PG balance in the body in favour of 1-series PGs. Particular diseases and disorders have been listed earlier herein.

The structure of spironolactone is

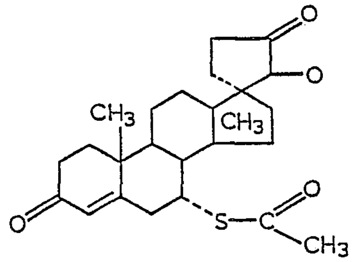

APPROACH TO PRESENT INVENTION

The approach to the present invention has been in part through the inventor's concern with zinc among other materials, shown in earlier work of his to be significant to PG metabolism. This work is discussed for example in European Patent Applications Nos. 79300079.5 and 79300546.3 (Publication Nos. 0003407 and 0004770).

Spironolactone was investigated for the reason that as a side effect it causes breast growth, and prolactin, which also causes breast growth, has a zinc-like action on PGE 1 biosynthesis.

EXPERIMENTAL RESULTS

In the case of spironolactone the effect of enhancement of 1-series PG production is demonstrated in results in human platelets and rat kidney slices given below.

Spironolactone is of particular interest in that it not only raises 1-series PG production from DGLA but at the same time reduces 2-series PG production from AA.

In the work, the detailed procedure of which is given later, human platelets and rat kidney slices were incubated with 14C-labelled arachidonic acid (AA) and dihomo-γ-linolenic acid (DGLA) in the presence or absence of 5 μg/ml spironolactone. The conversions of the precursors to the respective PGEs and TXBs in platelets and to the PGEs in kidney slices were investigated. The results are shown in the following tables and are expressed as percentages of the results obtained during control incubations when drug vehicle alone was added. A 10% variation is a reliable indication of real change.

TABLE 1

Kidney slices (rat)

| | Precursor acid<br>Product | DGLA<br>PGE 1 | AA<br>PGE 2 |
|---|---|---|---|
| 5 μg/ml spironolactone | | 143% | 67% |

TABLE 2

Platelets (human)

| | Precursor acid<br>Product | DGLA<br>PGE 1 | DGLA<br>TXB 1 | AA<br>PGE 2 | AA<br>TXB 2 |
|---|---|---|---|---|---|
| 5 μg/ml spironolactone | | 145% | 136% | 72% | 64% |

It is concluded that spironolactone both increases formation of 1-series products from DGLA and at the same time reduces formation of 2-series products from AA. The compound therefore influences the balance of 1-series and 2-series PGs in favour of 1-series PGs.

MATERIALS AND METHODS

The detailed technique with platelets is given below by way of example. That with kidney slices was essentially similar.

(1-$^{14}$C) arachidonic acid and (1-$^{14}$C) dihomo-γ-linolenic acid was used, diluted with hexane to specific activities of about 2 or 5 μCi/μmol. One day expired (2 days old) human platelets were obtained and used within 48 hours of expiration. One unit was centrifuged at 1000g for 15 minutes and the supernatant drawn off. The platelet pellet was resuspended in Tris-NaCl-EDTA buffer, made up of 0.15 M NaCl, 0.15 M Tris HCl at pH 7.4 and 0.007 M NaEDTA (90:8:2 v/v/v). The platelets were recentrifuged, the supernatant removed and the pellet was resuspended in Krebs-Henseleit buffer (without calcium) at oH 7.4. The washed platelet suspension contained about 1—2% red blood cells. All glassware used in the preparation of the platelets were siliconized.

Four equal sized 1 ml aliquots of the platelet suspension, containing $10^9$ platelets/ml were incubated with e.g. 0.5 μCi $^{14}$C-DGLA for five minutes. At the beginning of the incubation the drug under test was added to the suspension. The reaction was stopped after five minutes by addition of 1/10 volumes of 10% formic acid. The suspension was then extracted three times with ethyl acetate and the fractions pooled and

dried under vacuum. The extract was then taken up with 5 ml chloroform/methanol (2/1, v/v). Recovery of radioactive material in the extract was checked by taking 50 µl of the chloroform/methanol and counting by liquid scintillation. Recovery was in the range 80—95% in most experiments.

The chloroform/methanol extract was then reduced in volume to 1 ml under dry prepurified nitrogen. Thin layer chromatography was carried out on 500 µg precoated, prescored silica gel G Uniplates (Analtech). Plates were activated by heating to 100°C for 1 hour immediately prior to use. The solvent system was chloroform:methanol:acetic acid:water (90:8:1:0.8). Reference compounds (PG's El and Flα, and thromboxane Bl) were run at the same time and visualized by phosphomolybdic acid spray followed by brief heating. The bands on the plates corresponding to the reference PGE 1, PFG 1α and TXB 1 were scraped off and eluted with 20 ml acetone. Each elution was then evaporated to dryness and counted by liquid scintillation (Beckman 100 LS counter).

Using the same bath of platelets at the same time exactly similar experiments were carried out with $^{14}$-C-AA and PGE 2, PGE 2α and TXB 2 as reference compounds. Three experiments were performed with DGLA and three with AA.

RELATIONSHIP TO PREVIOUS PROPOSALS

The above approaches may be used in combination with other materials as disclosed in the earlier patent applications referred to above, to which reference may be made.

Among these materials are a number believed to act by enhancing mobilisation of DGLA reserves, including zinc, penicillin and β-lactam antibiotics generally, and also penicillamine, phenformin and levamisole when the other effects of these materials are acceptable.

Further, since there is evidence that thromboxane A2 may indirectly enhance formation of PGE 1, substances such as colchicine, amantadine and melatonin, as discussed in the pending patent applications and believed to act through increasing the production or effect of thromboxane A2, can also be used in conjunction with the materials of the present invention.

A further group of materials with which those of the present application may be used is disclosed in European Patent Application No. 80301510.6 (Publication No. 0019423), namely Vitamin C (ascorbic acid); ethyl alcohol; and naloxone, nalorphine, levallorphan and other opiate antagonists. These materials are discussed at length in that application, to which reference may be made and are a class that enhance physiological synthesis of 1-series PGs from DGLA without substantially enhancing synthesis of 2-series PGs from AA.

As appears from the earlier patent applications, in searching for ways to regulate PGE 1 formation the inventor has previously concentrated on the conversion of DGLA stores to free DGLA since this is believed to be a key rate-limiting step and since it has also been believed that factors which regulate conversion of free arachidonate to PGs will also regulate conversion of free DGLA to PGs. The present work has been more on the conversion of DGLA and of AA to the respective PGs, and as noted above it has been found that the factors regulating the two PG pathways are in some respects quite different. The discoveries on which the present application is based however build on and add to the earlier inventions, rather than superseding them.

DETAILED STATEMENT OF THE PRESENT INVENTION

In the light of the general discussion above the present invention may be summarised as a composition for treating any condition in which enhancement of 1-series PG production, or more broadly influence of the 1-series/2-series balance in the body in favour of 1-series PGs, is indicated, and particularly, any of the conditions listed earlier herein, which comprises (a) γ-linolenic acid and/or dihomo-γ-linolenic acid, optionally in association with linoleic acid and if desired other fat acids, said acids being used if desired as physiologically functional derivatives thereof, and (b) spironolactone or other modified steroid having its action.

DOSE RANGES (MATERIALS OF PRESENT INVENTION)

The following are effective:

| | |
|---|---|
| Spironolactone and other modified steroids | 30 mg to 2 g/day |

PACKS

If it is not desirable to have compositions comprising the active materials together, as listed above, packs may be prepared comprising the materials presented for separate or part joint and part separate administration in the appropriate relative amounts, and such packs are within the purview of the invention.

DIETARY COMPOSITIONS

The invention is chiefly described in terms of pharmaceutical compositions, but it will be understood that the γ-linolenic and other acids, being in the nature of dietary supplements, could be incorporated in a dietary margarine or other foodstuffs; such foodstuffs, possibly containing other active materials and generally referred to in this description as dietary or pharmaceutical compositions, are within the purview

of the invention and thus of the term pharmaceutical compositions, packs or the like used in the statement of invention and claims.

VETERINARY APPLICATIONS

It will be understood that where a disorder of a kind calling for treatment in animals arises, the invention while described primarily in terms of human medicine and treatment is equally applicable in the veternary field.

AMOUNTS OF ACTIVE MATERIALS (ADJUNCTS TO PRESENT INVENTION)

Amounts of materials are:

| | | |
|---|---|---|
| I | Zinc | 2.5 to 800 mg/day, preferably 10—80 mg, calculated as zinc |
| | β-lactam antibiotics | 0.5 to 10 g/day |
| | Penicillamine | 50 mg to 10 g/day |
| | Phenformin | 10 mg to 5 g/day |
| | Levamisole | 10 mg to 2 g/day |
| II | Colchicine | 0.3 to 15 mg/day, preferably 0.6 to 2.4 mg |
| | Melatonin | 10 mg to 5 g/day |
| | Amantadine | 100 mg to 1000 mg/day |
| | Griseofulvin | 0.5 g to 5 g/day |
| | Vinblastine | 35 mg to 350 mg/week |
| | Vincristine | 7 mg to 70 mg/week |
| | Interferon | $1 \times 10^5$ to $1 \times 10^8$ units/day |
| | Melatonin | 10 mg to 5 g/day |
| III | Ascorbic acid | 50 mg to 50 g preferably 250 mg to 5 g/day |
| | Ethyl alcohol | 5 ml to 500 ml preferably 50 ml to 200 ml/day |
| | Naloxone | 0.1 mg to 500 mg preferably 10 mg to 200 mg/day |
| | Nalorphine | 1 mg to 5 g preferably 10 mg to 2 g/day |
| | Levallorphan | 0.2 mg to 1 g preferably 10 mg to 1 g/day |

and comparable amounts of other opiate antagonists.

Detailed discussion of suitable amounts and forms of use is contained in the published patent applications referred to earlier to which reference may be made. In particular the β-lactam antibiotics are conveniently any of the known penicillin and cephalosporin antibiotics (including semi-synthetic antibiotics) such as, for example, penicillin G, penicillin N, penicillin V, cephalexin, cephalothin, ampicillin, amoxycillin, cloxacillin and cephaloglycin. Any of these may be used in the form of their physiologically functional non-toxic derivatives, for example alkali metal salts e.g. sodium and potassium salts, and salts with organic bases, and reference to an antibiotic herein includes reference to such derivatives.

AMOUNTS OF γ-LINOLENIC AND OTHE ACIDS SPECIFICALLY

A preferred daily dosage for all purposes for an adult (weight ca 75 kg) is from 0.05 to 0.1 up to 1, 2, 5 or even 10 g as required of γ-linolenic acid or equivalent weight calculated as γ-linolenic acid or a physiologically functional derivative thereof. Amounts in particular may be 0.1 to 1.0 g daily. Such doses correspond to about 2 to 20 g daily of the Oenothera oil discussed below. In place of, or in addition to, γ-linolenic acid, one may use dihomo-γ-linolenic acid or a physiologically functional derivative thereof, in amounts equivalent in molar tems to γ-linolenic acid and calculated as such. This dosage can for example be taken as a single dose or divided into 2, 3 or 4 subdivisions thereof as convenient.

FORMS AND SOURCES OF γ-LINOLENIC AND OTHER ACIDS

Convenient physiologically functional derivatives of γ-linolenic acid and dihomo-γ-linolenic acid for use according to the invention for all the purposes described include the $C_1$—$C_4$ alkyl (e.g. methyl esters and the glycerides of the acids).

If desired, pharmaceutical compositions may be produced for use in the invention by associating natural or synthetic γ-linolenic acid (or a physiologically functional derivative thereof) and/or dihomo-γ-linolenic acid (or a physiologically functional derivative thereof), as such, with an acceptable pharmaceutical vehicle. It is at present convenient to incorporate the γ-linolenic acid into compositions in the form of an available oil having a high γ-linolenic acid content, hence references to "oil" herein.

At the present time known natural sources of oils having a high γ-linolenic acid content are few (there are no known natural sources of significant amounts of dihomo-γ-linolenic acid). One source of oils currently available is the seed of Evening Primrose species such as *Oenothera biennis L.* and *Oenothera lamarckiana,* the oil extract therefrom containing γ-linolenic acid (about 8%) and linolenic acid (about 72%) in the form of their glycerides together with other glycerides (percentages based on total fatty acids). Another source of γ-linolenic acid is Borage species such as *Borago officinalis* which, though its current yield per acre is low, provides a richer source of γ-linolenic acid than Oenothera oil. Recent studies on fungi which can be cultivated by fermentation promise a fungal oil source.

The seed oil extracts referred to above can be used as such or can for example if desired be fractionated to yield an oily composition containing the triglycerides of γ-linolenic and linolenic as the main

fatty acid components, the γ-linolenic acid content being if desired a major proportion. Seed oil extracts appear to have a stablising effect upon any dihomo-γ-linolenic acid or physiologically functional derivative thereof.

PHARMACEUTICAL PRESENTATION

The compositions according to the invention are conveniently in a form suitable for oral, rectal, parenteral or topical administration in a suitable pharmaceutical vehicle, as discussed in detail for example in U.K. Patent Specification No. 1 082 624, to which reference may be made, and in any case very well known generally for any particular kind of preparation. Thus for example tablets, capsules, ingestible liquid or powder preparations, creams or lotions for topical application, or suppositories, can be prepared as required. Injectable solutions of hydrolysed Oenothera oil may be prepared using albumin to solubilise the free acid.

Advantageously a preservative is incorporated into the preparations. α-Tocopherol in a concentration of about 0.1% by weight has been found suitable for the purpose.

It will be understood that the absolute quantity of active ingredients present in any dosage unit should not exceed that appropriate to the rate and manner of administration to be employed but on the other hand should also desirably be adequate to allow the desired rate of administration to be achieved by a small number of doses. The rate of administration will moreover depend on the precise pharmacological action desired.

The following Examples serve to illustrate pharmaceutical compositions useful in treatment according to the invention.

Examples

Pharmaceutical compositions contain a unit dose of an oil extract from the seeds of *Oenothera biennis L.* and one of the active materials of the present invention, optionally with methyl dihomo-γ-linolenate and/or zinc oleate and/or penicillin V and/or any of the other active materials referred to herein directly or by cross reference to other patent applications of the inventor. They may be presented by encapsulation of the natural oil in soft gelatin capsules by known methods.

The oil is extracted from the seeds by one of the conventional methods of extraction such as cold pressure, screw pressure after partially cooking the seed, or solvent extraction.

Fractionation of a typical sample of this oil shows a yield of 97.0% oil in the form of methyl esters, with the relative proportions:

| | |
|---|---|
| Palmitate | 6.15 |
| Stearate | 1.6 |
| Oleate | 10.15 |
| Linoleate | 72.6 |
| γ-Linolenate | 8.9 |

As preservative, α-tocopherol is added to the oil in a concentration of 0.1%.

Gelatin capsules containing oil extracts prepared as described above, each having the following contents of active ingredients (0.5 g oil extract — ca 0.045 g γ-linolenic acid ), are prepared in conventional fashion.

The following is a specific example of capsules that may be given, two capsules three times a day, in treatment of the conditions listed earlier.

Example

Capsules containing:

| | |
|---|---|
| Oil extract | 0.5 g |
| Spironolactone | 50 mg |

Similarly capsules containing additional materials may be administered, for example 10 mg methyl dihomo-γ-linolenate per capsule as a direct supplement to the oil; or for example for their indirect action zinc oleate 10 mg or penicillin V 0.25 g (compare the Examples of European Patent Application No. 79300079.5 referred to earlier); or phenformin 25 mg, levamisole 25 mg or penicillamine 100 mg per capsule, or colchicine 0.2 mg, amantadine 100 mg or griseofulvin 0.5 mg per capsule (compare the Examples of European Patent Application No. 79300546.3 referred to earlier and also the use in conjunction with vincristine, vinblastine, melatonin and interferon referred to therein; or ascorbic acid 100 mg, naloxone 5 mg, nalorphine 5 mg or levallorphan 5 mg (compare the Examples of European Patent

Application No. 80301510.6 referred to earlier and also the use in conjunction with ethyl alcohol to give 30 to 300 mg% alcohol in the body referred to therein).

It will be understood throughout that while a full theoretical discussion of what is believed to be the reason for the effectiveness of the compositions proposed is given to aid understanding, the invention is in no way to be limited in this discussion.

**Claims for the Contracting States: BE CH DE FR GB IT LI LU NL SE**

1. A pharmaceutical composition comprising γ-linolenic acid and/or dihomo-γ-linolenic acid, or a salt, ester or other derivative convertible in the body thereto together with a compound influencing the 1-series/2-series PG balance in the body in favour of 1-series PGs and selected from spironolactone and compounds which have a steroid ring structure and which when tested on human platelets enhanced the conversion of DGLA (5,8,11-eicosatrienoic acid) to 1-series prostaglandins having its action alone or in an acceptable pharmaceutical vehicle.

2. A composition according to claim 1, presented for administration in quantities to give doses of 30 mg to 2g/day of said steroid.

3. A composition according to claim 1, presented for administration in quantities to give 0.05 to 10 g/day of said γ-linolenic or dihomo-γ-linolenic acid or derivative, calculated as γ-linolenic acid.

4. A composition according to claim 1, further comprising physiologically assimilable zinc, a β-lactam antibiotic, penicillamine, phenformin or levamisole.

5. A composition according to claim 4, presented for administration in quantities to give 2.5 to 800 mg/day zinc, 0.5 to 10 g/day β-lactam antibiotic, 50 mg to 10 g/day penicillamine, 10 mg to 5 g/day phenformin or 10 mg to 2 g/day levamisole.

6. A composition according to claim 1 further comprising a material selected from colchicine, griseofulvin, amantadine, melatonin, interferon, and vinblastine, vincristine and other Vinca alkaloids.

7. A composition according to claim 6, presented for administration in quantities to give doses of:

0.3 to 15 mg/day colchicine
100 to 1000 mg/day amantadine
0.5 to 5 g/day griseofulvin
35 to 350 mg/week vinblastine
7 to 70 mg/week vincristine
$1 \times 10^5$ to $1 \times 10^8$ units/day interferon, or
10 mg to 5 g/day melatonin.

8. A composition according to claim 1, further comprising a material selected from Vitamin C, ethyl alcohol, and naloxone, nalorphine, levallorphan or other opiate antagonist.

9. A composition according to claim 8, presented for administration in quantities to give doses of:

50 mg to 50 g/day ascorbic acid or
5 to 500 ml/day ethyl alcohol or
0.1 to 500 mg/day naloxone, 1 mg to 5 g/day nalorphine, 0.2 mg to 1 g/day levallorphan or comparable amount of other opiate antagonist.

10. A composition according to any preceding claim, when for use in therapy for influencing the 1-series/2-series PG balance in the body in favour of 1-series PGs.

11. A pharmaceutical pack comprising the materials set out in any preceding claim presented separately, or one or more separately and other together, but for conjoint administration.

**Claims for the Contracting State: AT**

1. The use of γ-linolenic acid and/or dihomo-γ-linolenic acid, or a salt, ester or other derivative convertible in the body thereto together with a compound influencing the 1-series/2-series PG balance in the body in favour of 1-series PGs and selected from spironolactone and compounds which have a steroid ring structure and which when tested on human platelets enhanced the conversion of DGLA (5,8,11-eicosatrienoic acid) to 1-series prostaglandins alone or in an acceptable pharmaceutical vehicle for the method of producing a pharmaceutical composition for influencing the 1-series/2-series PG balance in the body in favour of 1-series PGs.

2. The use of claim 1, the composition being presented for administration in quantities to give doses of 30 mg to 2 g/day of said steroid.

3. The use of claim 1, the composition being presented for administration in quantities to give 0.05 to 10 g/day of said γ-linolenic acid or dihomo-γ-linolenic acid or derivative, calculated as γ-linolenic acid.

4. The use of claim 1, the composition further comprising physiologically assimilable zinc, a β-lactam antibiotic, penicillamine, phenformin or levamisole.

5. The use of claim 4, the composition being presented for administration in quantities to give 2.5 to

800 mg/day zinc, 0.5 to 10 g/day β-lactam antibiotic, 50 mg to 10 g/day penicillamine, 10 mg to 5 g/day phenformin or 10 mg to 2 g/day levamisole.

6. The use of claim 1, the composition being further comprising a material selected from colchicine, griseofulvin, amantadine, melatonin, interferon, and vinblastine, vincristine and other Vinca alkaloids.

7. The use of claim 6, the composition being presented for administration in quantities to give doses of:

0.3 to 15 mg/day colchicine
100 to 1000 mg/day amantadine
0.5 to 5 g/day griseofulvin
35 to 350 mg/week vinblastine
7 to 70 mg/week vincristine
$1 \times 10^5$ to $1 \times 10^8$ units/day interferon, or
10 mg to 5 g/day melatonin.

8. The use of claim 1, the composition further comprising a material selected from Vitamin C, ethyl alcohol, and naloxone, nalorphine, levallorphan or other opiate antagonist.

9. The use of claim 8, the composition being presented for administration in quantities to give doses of:

50 mg to 50 g/day ascorbic acid or
5 to 500 ml/day ethyl alcohol or
0.1 to 500 mg/day naloxone, 1 mg to 5 g/day nalorphine, 0.2 mg to 1 g/day levallorphan or comparable amounts of other opiate antagonist.

**Patentansprüche für die Vertragsstaaten: BE CH DE FR GB IT LI LU NL SE**

1. Pharmazeutisches Präparat, enthaltend γ-Linolensäure und/oder Dihomo-γ-linolensäure oder deren im Körper in diese überführbares Salz, Ester bzw. sonstiges Derivat, zusammen mit einer Verbindung, die das Prostaglandingleichgewicht zwischen der 1-Serie und 2-Serie im Körper zugunsten der 1-Serie beeinflusst und unter Spironolacton und Verbindungen ausgewählt ist, die eine Steroidringkonstitution besitzen und bei der Prüfung an menschlichen Thrombocyten die Umwandlung von Dihomo-γ-linolensäure (5,8,11-Eikosatriensäure) zu Prostaglandinen der 1-Serie fördern, wobei das Präparat für sich oder in einem unbedenklichen pharmazeutischen Träger wirksam ist.

2. Präparat nach Anspruch 1, konfektioniert zur Verabreichung in Dosierungen von 30 mg bis 2 g/Tag besagten Steroids ergebenden Mengen.

3. Präparat nach Anspruch 1, konfektioniert zur zur Verabreichung in 0,05 bis 10 g/Tag besagter γ-Linolen- oder Dihomo-γ-linolensäure bzw. deren Derivats, berechnet als γ-Linolensäure, ergebenden Mengen.

4. Präparat nach Anspruch 1, ferner enthaltend physiologisch assimilierbares Zink, ein β-Lactam-Antibiotikum, Penicillamin, Phenformin oder Levamisol.

5. Präparat nach Anspruch 4, konfektioniert zur Verabreichung in 2,5 bis 800 mg/Tag Zink, 0,5 bis 10 g/Tag β-Lactam-Antibiotikum, 50 mg bis 10 g/Tag Penicillamin, 10 mg bis 5 g/Tag Phenformin oder 10 mg bis 2 g/Tag Levamisol ergebenden Mengen.

6. Präparat nach Anspruch 1, ferner enthaltend einen unter Colchicin, Griseofulvin, Amantadin, Melatonin, Interferon sowie Vinblastin, Vincristin und anderen Vincaalkaloiden ausgewählten Stoff.

7. Präparat nach Anspruch 6, konfektioniert zur Verabreichung in Dosierungen von

0,3 bis 15 mg/Tag Colchicin
100 bis 1000 mg/Tag Amantadin
0,5 bis 5 g/Tag Grisofulvin
35 bis 350 mg/Woche Vinblastin
7 bis 70 mg/Woche Vincristin
$1 \,.\, 10^5$ bis $1 \,.\, 10^8$ Einheiten pro Tag Interferon oder
10 mg bis 5 g/Tag Melatonin

ergebenden Mengen.

8. Präparat nach Anspruch 1, ferner enthaltend einen unter Vitamin C, Aethylalkohol sowie Naloxon, Nalorphin, Levallorphan oder einem anderen Opiatantagonisten ausgewählten Stoff.

9. Präparat nach Anspruch 8, konfektioniert zur Verabreichung in Dosierungen von

50 mg bis 50 g/Tag Ascorbinsäure oder
5 bis 500 ml/Tag Aethylalkohol oder
0,1 bis 500 mg/Tag Naloxon, 1 mg bis 5 g/Tag Nalorphin, 0,2 mg bis 1 g/Tag Levallorphan oder einen vergleichbaren Betrag eines anderen Opiatantagonisten

ergebenden Mengen.

10. Präparat nach einem der vorhergehenden Ansprüche, wenn therapeutisch zur Beeinflussung des Prostaglandingleichgewichts zwischen der 1-Serie und 2-Serie im Körper zugunsten der 1-Serie verwendet.

11. Pharmazeutische Packung, enthaltend die in einem der vorhergehenden Ansprüche angegebenen Stoffe, jeweils getrennt oder einer oder mehrere getrennt mit anderen zusammen, jedoch zur gemeinsamen Verabreichung.

**Patentansprüche für den Vertragsstaat: AT**

1. Verwendung von γ-Linolensäure und/oder Dihomo-γ-linolensäure oder einem im Körper in diese überführbaren Salz, Ester oder sonstigen Derivat, zusammen mit einer Verbindung, die das Prostaglandingleichgewicht zwischen der 1-Serie und 2-Serie im Körper zugunsten der 1-Serie beeinflusst und unter Spironolacton und Verbindungen ausgewählt ist, die eine Steroidringkonstitution besitzen und bei der Prüfung an menschlichen Thromobocyten die Umwandlung von Dihomo-γ-linolensäure (5,8,11-Eikosatriensäure) zu Prostaglandinen der 1-Serie förden, für sich oder in einem unbedenklichen pharmazeutischen Träger, für das Verfahren zur Herstallung eines pharmazeutischen Präparats zur Beeinflussung des Prostaglandingleichgewichts zwischen der 1-Serie und 2-Serie im Körper zungunsten der 1-Serie.

2. Verwendung nach Anspruch 1, wobei das Präparat zur Verabreichung in Dosierungen von 30 mg bis 2 g/Tag besagten Steroids ergebenden Mengen konfektioniert ist.

3. Verwendung nach Anspruch 1, wobei das Präparat zur Verabreichung in 0,05 bis 10 g/Tag besagter γ-Linolen- oder Dihomo-γ-linolensäure bzw. deren Derivats, berechnet als γ-Linolensäure, ergebenden Mengen konfektioniert ist.

4. Verwendung nach Anspruch 1, wobei das Präparat ferner physiologisch assimilierbares Zink, ein β-Lactam-antibiotikum, Penicillamin, Phenformin oder Levamisol enthält.

5. Verwendung nach Anspruch 4, wobei das Präparat zur Verabreichung in 2,5 bis 800 mg/Tag Zink, 0,5 bis 10 g/Tag β-Lactam-antibiotikum, 50 mg bis 10 g/Tag Penicillamin, 10 mg bis 5 g/Tag Phenformin oder 10 mg bis 2 g/Tag Levamisol ergebenden Mengen konfektioniert ist.

6. Verwedung nach Anspruch 1, wobei das Präparat ferner einen unter Colchicin, Griseofulvin, Amantadin, Melatonin, Interferon sowie Vinblastin, Vincristin und anderen Vinca-alkaloiden ausgewählten Stoff enthält.

7. Verwendung nach Anspruch 6, wobei das Präparat zur Verabreichung in Dosierungen von
  0,3 bis 15 mg/Tag Colchicin
  100 to 1000 mg/Tag Amantadin
  0,5 bis 5 g/Tag Griseofulvin
  35 bis 350 mg/Woche Vinblastin
  7 bis 70 mg/Woche Vincristin
  $1 . 10^5$ bis $1 . 10^8$ Einheiten pro Tag Interferon oder 10 mg bis 5 g/Tag Melatonin
  ergebenden Mengen kongektioniert ist.

8. Verwendung nach Anspruch 1, wobei das Präparat ferner einen unter Vitamin C, Aethylalkohol sowie Naloxon, Nalorphin, Levallorphan oder einem anderen Opiatantagonisten ausgewählten Stoff enthält.

9. Verwendung nach Anspruch 8, wobei das Präparat zur Verabreichung in Dosierungen von
  50 mg bis 50 g/Tag Ascorbinsäure oder
  5 bis 500 ml/Tag Aethylalkohol oder
  0,1 bis 500 mg/Tag Naloxan, 1 mg bis 5 g/Tag Nalorphin, 0,2 mg bis 1 g/Tag Levallorphan oder einen vergleichbaren Betrag eines anderen Opiatantagonisten
ergebenden Mengen konfektioniert ist.

**Revendications pour les Etats contractants: BE CH DE FR GB IT LI LU NL SE**

1. Composition pharmaceutique comprenant l'acide γ-linolénique et/ou l'acide dihomo-γ-linolénique ou un sel, ester ou autre dérivé convertible en ce produit, ainsi qu'un composé influençant l'équilibre PG série 1/série 2 dans le corps en faveur des PG de la série 1 et choisi parmi la spironolactone et les composés qui possèdent une structure stéroïde cyclique et qui, lorsqu'ils sont testés sur des plaquettes humaines, favorisent la conversion du DGLA (acide 5,8,11-éicosatriénoïque) en prostaglandines de la série 1 et possédant son action seul ou dans un excipient pharmaceutique acceptable.

2. Composition selon la revendication 1, présentée pour l'administration en quantités convenables pour donner des doses de 30 mg à 2 g/jour du dit stéroïde.

3. Composition selon la revendication 1, présentée pour l'administration en quantités convenables pour donner, 0,05 à 10 g/jour du dit acide γ-linolénique dihomo-γ-linolénique ou de son dérivé, calculé en acide γ-linolénique.

4. Composition selon la revendication 1, comprenant en outre du zinc physiologiquement assimilable, un antibiotique β-lactamique, de la pénicillamine, de la phénformine ou de la lévamisole.

5. Composition selon la revendication 4, présentée pour l'administration en quantités convenables pour donner 2,5 à 800 mg/jour de zinc, 0,5 à 10 g/jour d'antibiotique β-lactamique, 50 mg à 10 g/jour de pénicillamine, 10 mg à 5 g/jour de phénformine ou 10 mg à 2 g/jour de lévamisole.

6. Composition selon la revendication 1, comprenant en outre un matériau choisi parmi la colchicine, la griséofulvine, l'amantadine, la mélatonine, l'interféron at la vinblastine, la vincristine et autres alcaloïdes de Vinca.

7. Composition selon la revendication 6, présentée pour l'administration en quantités convenables pour donner des doses de:
  0,3 à 15 mg/jour de colchicine
  100 à 1000 mg/jour d'amantadine

0,5 à 5 g/jour de griséofulvine
35 à 350 mg/semaine de vinblastine
7 à 70 mg/semaine de vincristine
$1 \times 10^5$ à $1 \times 10^8$ unités/jour d'interféron, ou 10 mg à 5 g/jour de mélatonine.

8. Composition selon la revendication 1, comprenant en outre un matériau choisi parmi la vitamine C, l'alcool éthylique at la naloxone, la nalorphine, la lévallorphane ou autre antagoniste opiacé.

9. Composition selon la revendication 8, présentée pour l'administration en quantités convenables pour donner des doses de:
50 mg à 50 g/jour d'acide ascorbique ou
5 à 500 ml/jour d'alcool éthylique ou
0,1 à 500 mg/jour de naloxone, 1 mg à 5 g/jour de nalorphine, 0,2 mg à 1 g/jour de lévallorphane ou une quantité comparable d'autre antagoniste opiacé.

10. Composition selon l'une quelconque des revendications précédentes, destinée à être employée dans le traitement pour influencer l'équilibre PG série 1/série 2 dans le corps en faveur des PG de la série 1.

11. Conditionnement pharmaceutique comprenant les matériaux définis dans les revendications précédentes présentés séparément, ou un ou plusieurs séparément et d'autres ensemble, mais destinés à être administrés conjointement.

**Revendications pour l'Etat contractant: AT**

1. Utilisation de l'acide γ-linolénique et/ou de l'acide dihomo-γ-linolénique, ou d'un sel, d'un ester ou autre dérivé convertible en ce produit, ainsi qu'un composé influençant l'équilibre PG série 1/série 2 dans le corps en faveur des PG de la série 1 et choisi parmi la spironolactone et les composés qui possèdent une structure stéroïde cyclique et qui, lorsqu'ils sont testés sur des plaquettes humaines, favorisent la conversion du DGLA (acide, 5,8,11-éicosatriénoïque) en prostaglandines de la série 1 et possédant son action seul ou dans un excipient pharmaceutique acceptable.

2. Utilisation selon la revendication 1, la composition étant présentée pour l'administration en quantités convenables pour donner des doses de 30 mg à 2 g/jour du dit stéroïde.

3. Utilisation selon la revendication 1, la composition étant présenté pour l'administration en quantités convenables pour donner 0,05 à 10 g/jour du dit acide γ-linolénique ou dihomo-γ-linolénique ou de son dérivé, calculé en acide γ-linolénique.

4. Utilisation selon la revendication 1, la composition comprenant en outre du zinc physiologiquement assimilable, un antibiotique β-lactamique, de la pénicillamine, de la phénformine ou de la lévamisole.

5. Utilisation selon la revendication 4, la composition étant présentée pour l'administration en quantités convenables pour donner 2,5 à 800 mg/jour de zinc, 0,5 à 10 g/jour d'antibiotique β-lactamique, 50 mg à 10 g/jouir de pénicillamine, 10 mg à 5 g/jour de phénformine ou 10 mg à 2 g/jour de lévamisole.

6. Utilisation selon la revendication 1, la composition comprenant en outre un matériau choisi parmi la colchicine, la griséofulvine, l'amantadine, la mélatonine, l'interféron et la vinblastine, la vincristine et autres alcaloïdes de Vinca.

7. Utilisation selon la revendication 6, la composition étant présentée pour l'administration en quantités convenables pour donner des doses de:
0,3 à 15 mg/jour de colchicine
100 à 1000 mg/jour d'amantadine
0,5 à 5 g/jour de griséofulvine
35 à 350 mg/semaine de vinblastine
7 à 70 mg/semaine de vincristine
$1 \times 10^5$ à $1 \times 10^8$ unités/jour d'interféron, ou 10 mg à 5 g/jour de mélatonine.

8. Utilisation selon la revendication 1, la composition comprenant en outre un matériau choisi parmi la vitamine C, l'alcool éthylique et la naloxone, la nalorphine, la lévallorphane ou autre antagoniste opiacé.

9. Utilisation selon la revendication 8, présentée pour l'administration en quantités convenables pour donner des doses de:
50 mg à 50 g/jour d'acide ascorbique ou
5 à 500 ml/jour d'alcool éthylique ou
0,1 à 500 mg/jour de naloxone, 1 mg à 5 g/jour de nalorphine, 0,2 mg à 1 g/jour de lévallorphane ou une quantité comparable d'autre antagoniste opiacé.